(19) Europäisches Patentamt
European Patent Office
Office européen des brevets

(11) EP 1 062 937 B1

(12) **FASCICULE DE BREVET EUROPEEN**

(45) Date de publication et mention de la délivrance du brevet:
**24.08.2005 Bulletin 2005/34**

(51) Int Cl.[7]: **A61K 7/13**, A61K 7/135, A61K 7/06

(21) Numéro de dépôt: **00401166.4**

(22) Date de dépôt: **27.04.2000**

(54) **Composition de teinture d'oxydation des fibres kératiniques et procédé de teinture mettant en oeuvre cette composition**

Oxidationsfärbemittel für keratinische Fasern und Färbungsverfahren mit diesem Mittel

Composition for oxidative dyeing of keratinous fibres and dyeing process using the same

(84) Etats contractants désignés:
**AT BE CH CY DE DK ES FI FR GB GR IE IT LI LU MC NL PT SE**

(30) Priorité: **21.06.1999 FR 9907828**

(43) Date de publication de la demande:
**27.12.2000 Bulletin 2000/52**

(73) Titulaire: **L'OREAL**
**75008 Paris (FR)**

(72) Inventeurs:
- **Plos, Grégory**
**75015 Paris (FR)**
- **Lagrange, Alain**
**77700 Coupvray (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 0 504 005**
**EP-A- 0 636 686**
**WO-A-96/29046**
**WO-A-99/15139**
**WO-A-99/20236**
**FR-A- 2 763 841**
**FR-A- 2 768 617**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 062 937 B1

Printed by Jouve, 75001 PARIS (FR)

## Description

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins un colorant d'oxydation, au moins une enzyme de type oxydo-réductase à 2 ou à 4 électrons, et au moins un chitosane particulier salifié ou chimiquement modifié, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes du type oxydo-réductases à 2 électrons, telles que la pyranose-oxydase, la glucose-oxydase ou bien l'uricase, en présence d'un donneur pour lesdites enzymes.

Il a déjà également été proposé, notamment dans les demandes de brevets FR-A-2112549, FR-A-2694018, EP-A-504005, WO 95/07988, WO95/33836, WO95/33837, WO96/00290, WO97/19998, WO97/19999 et brevet US-3251742, de teindre les fibres kératiniques avec des compositions comprenant notamment un précurseur de colorant d'oxydation et une enzyme de type laccase (oxydo-réductase à 4 électrons).

Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas entière satisfaction notamment du point de vue de leur intensité car on suppose que les épaississants généralement utilisés dans ce type de teinture aux enzymes freinent la montée de la couleur sur la fibre. En outre, l'oxygène moléculaire se dissout mal dans les supports classiques de teinture auxdites enzymes, ce qui a pour effet de diminuer l'activité des enzymes en teinture capillaire.

Les documents WO-A-99/20236, WO-A-99/15139, FR-A-2763841 et FR-A-2768617 divulgent des compositions de teinture d'oxydation contenant an moins un colorant d'oxydation, au moins une enzyme de type oxyde-réductase à 2 électrons et des agents épaississants.

Les documents EP-A-636686 et WO-A-96/29046, décrivent l'utilisation du chitosane dans des compositions de lavage et de teinture des cheveux.

Or, la demanderesse vient maintenant de découvrir qu'il est possible d'obtenir de nouvelles teintures, capables de conduire à des colorations plus intenses en associant au moins un colorant d'oxydation, au moins une enzyme de type oxydo-réductase à 2 électrons, ou à 4 électrons, et au moins un chitosane particulier, salifié ou chimiquement modifié.

Il est également possible d'obtenir une meilleure conservation de l'activité des enzymes utilisées en coloration capillaire.

Ces découvertes sont à la base de la présente invention.

L'invention a donc pour premier objet une composition prête à l'emploi, pour la teinture d'oxydation des fibres

kératiniques et en particulier des fibres kératiniques humaines telles que les cheveux, caractérisée par le fait qu'elle comprend, dans un milieu approprié pour la teinture :

- au moins un colorant d'oxydation,
- au moins une enzyme de type oxydo-réductase à 2 électrons ou à 4 électrons , et
- au moins un chitosane salifié par un acide organique ou minéral, permettant d'obtenir à 1% dans l'eau une solution visuellement limpide,
- ou un chitosane chimiquement modifié comprenant un ou plusieurs motifs de formule (I) suivante :

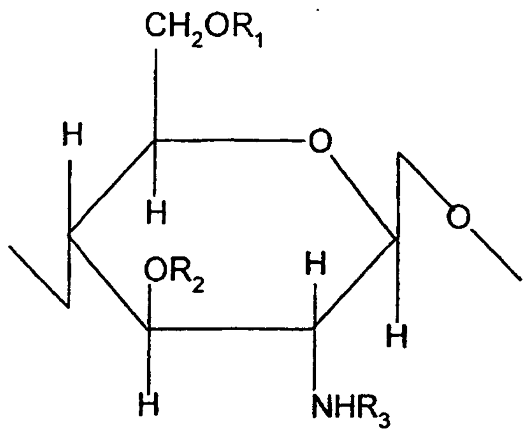

(I)

dans laquelle,

$R_1$, $R_2$, identiques ou différents, désignent un atome d'hydrogène ou un radical -XCOOM,

$R_3$ désigne un atome d'hydrogène, un radical $-COCH_3$, ou un radical -CO-X-COOM,

X représente un radical alkylène en $C_1$-$C_8$ éventuellement ramifié, ou substitué par un ou plusieurs groupements hydroxyle, halogène ou époxy,

M désigne un atome d'hydrogène ou un cation choisi parmi les métaux alcalins, alcalino-terreux, l'ammonium, une amine ou une alcanolamine organiques,

sous réserve que, l'un au moins des motifs de formule (I) comportant un radical $R_1$ et/ou $R_2$ et/ou $R_3$ désigne -XCOOM et/ou un radical $R_3$ désigne -CO-X-COOM,
ou un N,O-carboxyméthylchitosane.

L'invention a également pour objet un procédé de teinture d'oxydation des fibres kératiniques mettant en oeuvre cette composition tinctoriale prête à l'emploi.

Les chitosanes salifiés utilisés dans la composition tinctoriale prête à l'emploi conforme à l'invention peuvent notamment être choisis parmi ceux salifiés par un acide organique tels que par exemple l'acide lactique, l'acide glutamique et de préférence l'acide pyrrolidone carboxylique ou parmi ceux salifiés par un acide minéral tel que par exemple l'acide chlorhydrique et l'acide sulfurique, sous réserve qu'ils donnent à la concentration pondérale de 1% dans l'eau, une solution visuellement limpide.

Parmi les chitosanes chimiquement modifiés, on peut notamment citer le produit vendu sous la dénomination N,O-carboxymethylchitosan par la société CHITOGENICS LTD, le N-carboxybutylchitosane vendu sous les dénominations commerciales CHITOLAM NB 101 ou EVALSAN par la société CHITO BIOS , le N-succinylchitosane vendu par la société CHIMEX sous la dénomination MEXOMERE PAD, ou vendu par la société FRANCECHITINE sous la dénomination KITINAMI, ou vendu par la société KATAKURA CHIKKARIN sous la dénomination SUCCINYL CHITOSAN, le N-succinylcarboxyméthylchitosane vendu sous la dénomination CHITOSOLLEN par la société IKEDA.

Le ou les chitosanes salifiés ou modifiés de formule (I) utilisés dans la composition tinctoriale prête à l'emploi conforme à l'invention représentent de préférence de 0,01 à 20 % en poids environ par rapport au poids total de la composition tinctoriale prête à l'emploi et encore plus préférentiellement de 0,1 à 5 % en poids environ de ce poids.

La ou les oxydo-réductases à 2 électrons utilisées dans la composition tinctoriale prête à l'emploi conforme à l'invention, en présence d'un donneur pour la ou lesdites enzymes, peuvent notamment être choisies parmi les

pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition, et encore plus préférentiellement de 0,1 à 5 % en poids environ de ce poids.

On peut aussi définir la quantité d'enzyme en fonction de son activité.
L'activité enzymatique des oxydoréductases à 2 électrons conformes à l'invention peut être définie à partir de l'oxydation du donneur en condition aérobie.
Une unité U correspond à la quantité d'enzyme conduisant à la génération d'une µmole de $H_2O_2$ par minute à un pH de 8,5 et à une température de 25°C. De façon préférentielle, la quantité d'oxydoréductase à 2 électrons conforme à l'invention est comprise entre 10 et $10^8$ unités U environ pour 100g de composition tinctoriale.

Selon l'invention, on entend par donneur, les différents substrats également nécessaires au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose; à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; à titre de donneur pour les uricases, on peut citer l'acide urique et ses sels ; à titre de donneur pour les choline oxydases, on peut citer la choline et ses sels d'addition avec un acide comme le chlorhydrate de choline, et la bétaïne aldéhyde ; à titre de donneur pour les sarcosine oxydases, on peut citer la sarcosine, la N-méthyl-L-leucine, la N-méthyl-DL-alanine, et la N-méthyl-DL-valine ; et enfin, à titre de donneur pour les bilirubine oxydases, on peut citer la bilirubine.

Le ou les donneurs (ou substrats) utilisés conformément à l'invention représentent de préférence de 0,01 à 20 % en poids environ du poids total de la composition conforme à l'invention et encore plus préférentiellement de 0,1 à 5 % en environ de ce poids.

La ou les oxydo-réductases à 4 électrons utilisées dans la composition tinctoriale prête à l'emploi conforme à l'invention peuvent notamment être choisies parmi les laccases, les tyrosinases, les catéchol oxydases et les polyphénols oxydases.

Selon une forme de réalisation particulière et préférée de l'invention la ou les oxydo-réductases à 4 électrons sont choisies parmi les laccases.

Ces laccases peuvent notamment être choisies parmi les laccases d'origine végétale, d'origine animale, d'origine fongique (levures, moisissures, champignons) ou d'origine bactérienne, les organismes d'origine pouvant être mono- ou pluricellulaires. Les laccases peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.

On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena

unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.

L'activité enzymatique des laccases utilisées conformément à l'invention et ayant la syringaldazine parmi leurs substrats peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5.
L'activité enzymatique des laccases utilisées selon l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

De manière générale, la ou les oxydo-réductases à 4 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

De manière particulière, et lorsqu'une ou plusieurs laccases sont utilisées, la quantité de laccase(s) présente dans la composition tinctoriale prête à l'emploi conforme à l'invention variera en fonction de la nature de la ou des laccases utilisées. De façon préférentielle, la quantité de laccase(s) est comprise entre 0,5 et 2000 Lacu environ (soit entre 10000 et $40.10^6$ unités U environ ou soit entre 20 et $20.10^6$ unités ulac) pour 100 g de composition tinctoriale prête à l'emploi.

Le ou les colorants d'oxydation utilisés dans la composition tinctoriale prête à l'emploi conforme à l'invention peuvent notamment être choisis parmi des bases d'oxydation et/ou des coupleurs.
Les bases d'oxydation sont notamment les paraphénylènediamines, les bases doubles, les para-aminophénols et les bases hétérocycliques.

A titre d'exemples, on peut citer parmi les paraphénylènediamines, celles de formule (II) suivante, et leurs sels d'addition avec un acide :

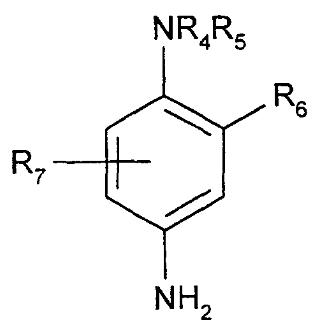

(II)

dans laquelle :

- $R_4$ et $R_5$, identiques ou différents, représentent un atome d'hydrogène, un radical alkyle en $C_1$-$C_4$, ou monohydroxyalkyle en $C_1$-$C_4$ ;
- $R_6$ représente un atome d'hydrogène ou d'halogène, un radical alkyle en $C_1$-$C_4$, ou monohydroxyalkyle en $C_1$-$C_4$ ;
- $R_7$ représente représentent un atome d'hydrogène ou un radical alkyle en $C_1$-$C_4$ ;

Parmi les paraphénylènediamines de formule (II) ci-dessus, on peut plus particulièrement citer :
la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxy-éthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, et leurs sels d'addition avec un acide.

Parmi les bases doubles utilisables à titre de base d'oxydation dans la composition tinctoriale prête à l'emploi

conforme à l'invention, on peut notamment citer les composés comportant au moins deux noyaux aromatiques sur lesquels sont portés plusieurs groupements amino et/ou hydroxyle.

Parmi lesdites bases doubles on peut plus particulièrement citer les composés de formule (III) suivante, et leurs sels d'addition avec un acide :

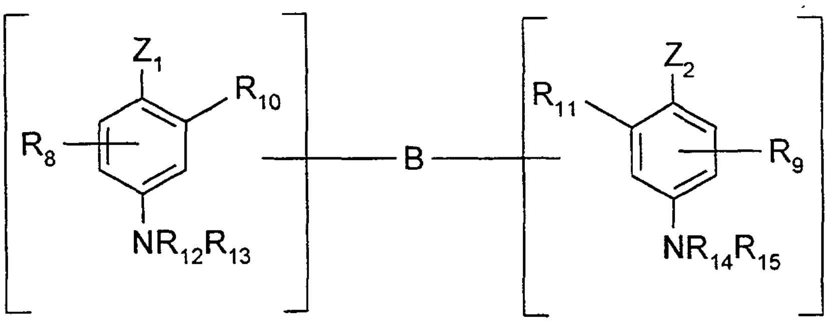

(III)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y;
- le bras de liaison B représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_8$ et $R_9$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{10}$, $R_{11}$, $R_{12}$, $R_{13}$, $R_{14}$ et $R_{15}$, identiques ou différents, représentent un atome d'hydrogène, un bras de liaison B ou un radical alkyle en $C_1$-$C_4$ ;

étant entendu que les composés de formule (III) ne comportent qu'un seul bras de liaison B par molécule.

Parmi les groupements azotés de la formule (III) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formule (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de base d'oxydation dans la composition tinctoriale prête à l'emploi conforme à l'invention, on peut notamment citer ceux de formule (IV) suivante et leurs sels d'addition avec un acide :

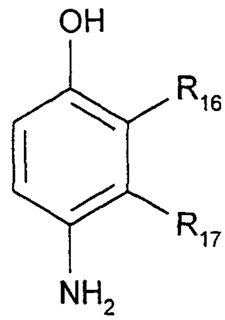

(IV)

dans laquelle :

- $R_{16}$ et $R_{17}$, identiques ou différents, représentent un atome d'hydrogène ou d'halogène, un radical alkyle en $C_1$-$C_4$, monohydroxyalkyle en $C_1$-$C_4$, alcoxy($C_1$-$C_4$)alkyle en $C_1$-$C_4$, aminoalkyle en $C_1$-$C_4$, monohydroxyalkyle($C_1$-$C_4$) aminoalkyle en $C_1$-$C_4$,

étant entendu qu'au moins un des radicaux $R_{16}$ et $R_{17}$ représente un atome d'hydrogène ;

Parmi les para-aminophénols de formule (IV) décrite ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les bases hétérocycliques utilisables à titre de base d'oxydation dans la composition tinctoriale prête à l'emploi conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Les coupleurs, sont notamment les méta-aminophénols, les métaphénylènediamines, les métadiphénols, les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, et leurs sels d'addition avec un acide.

Ces coupleurs sont plus particulièrement choisis parmi le 2-méthyl 5-amino phénol, le 5-N-(β-hydroxyéthyl) amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'a-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Selon l'invention, le ou les colorants d'oxydation représentent de préférence de 0,001 à 20 % en poids environ du poids total de la composition tinctoriale prête à l'emploi, et encore plus préférentiellement de 0,01 à 10 % en poids environ de ce poids.

Selon une forme de réalisation préférée, la composition tinctoriale prête à l'emploi conforme à l'invention peut en outre renfermer un ou plusieurs colorants directs notamment pour modifier les nuances en les enrichissant de reflets.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale prête à l'emploi, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons ou à 4 électrons soit suffisante. Il est généralement compris entre 3 et 11 environ, et de préférence entre 4 et 9 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino 1-propanol ainsi que leurs dérivés, les hydroxydes de sodium ou de potassium et les composés de formule (V) suivante :

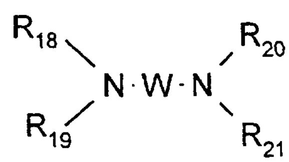

(V)

dans laquelle W est un reste propylène éventuellement substitué par un groupement hydroxyle ou un radical alkyle en $C_1$-$C_4$ ; $R_{18}$, $R_{19}$, $R_{20}$ et $R_{21}$, identiques ou différents, représentent un atome d'hydrogène, un radical alkyle en $C_1$-$C_4$ ou hydroxyalkyle en $C_1$-$C_4$.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses,

telles que sous forme de liquides, de crèmes, de gels, éventuellement pressurisés, ou sous toute autre forme appropriée pour réaliser une teinture des fibres kératiniques, et notamment des cheveux humains. Dans ce cas, les colorants d'oxydation et la ou les oxydo-réductases à 2 électrons ou à 4 électrons sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres, à une température d'application comprise entre la température ambiante et 80°C, au moins une composition tinctoriale prête à l'emploi telle que définie précédemment, pendant un temps suffisant pour développer la coloration désirée. De façon préférentielle, les fibres sont ensuite rincées, on éventuellement lavées au shampooing, puis séchées.

La température d'application est de préférence comprise entre la température ambiante et 60°C et encore plus préférentiellement entre 35°C et 50°C.

Le temps suffisant au développement de la coloration sur les fibres kératiniques est généralement compris entre 1 et 60 minutes et encore plus précisément entre 5 et 30 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation ( base et/ou coupleur) telle que défini précédemment, et d'autre part, une composition (B) renfermant, dans un milieu approprié pour la teinture, au moins une enzyme de type oxydo-réductase à 2 électrons ou à 4 électrons, ladite composition (A) et/ou ladite composition (B) renfermant au moins un chitosane salifié ou chimiquement modifié tel que défini ci-dessus, puis à procéder à leur mélange au moment de l'emploi avant d'appliquer ce mélange sur les fibres kératiniques.

Un autre objet de l'invention est un dispositif de teinture à plusieurs compartiments ou "kit" de teinture ou tout autre système de conditionnement à plusieurs compartiments dont au moins un premier compartiment renferme la composition (A) telle que définie ci-dessus et au moins un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

EXEMPLE 1

On a comparé une composition de teinture B selon l'invention contenant comme épaississant un N,O-carboxyméthyl chitosane à une composition A de l'art antérieur contenant comme épaississant un terpolymère acide méthacrylique/acrylate d'éthyle/méthacrylate de stéaryle oxyéthyléné en dispersion aqueuse à 30% (Aculyn 22 de Rohm and Haas) dont les compositions sont données ci-après.
Chacune des compositions a été appliquée sur des mèches de cheveux gris naturels à 90% de blancs, pendant 30 minutes à 40°C.
A l'issue du temps de pause, les mèches de cheveux ont été rincées, lavées avec un shampooing, puis séchées.

La couleur a ensuite été mesurée au colorimètre MINOLTA CM2002 dans le système L* a* b* .
Dans le système L* a* b* les 3 paramètres désignent respectivement l'intensité (L* ), la nuance (a* ), et la saturation (B* ).
Selon ce système, plus la valeur de L est élevée, plus la couleur est claire ou peu intense. Inversement, plus la valeur de L est faible, plus la couleur est foncée ou très intense.
a* et b* indiquent deux axes de couleurs, a* indique l'axe de couleur vert/rouge et b* l'axe de couleur bleu/jaune. Des valeurs proches de zéro pour a* et b* correspondent à des nuances grises.
La montée de la coloration ΔE peut être calculée en appliquant l'équation suivante :

$$\Delta E = \sqrt{(L^*-L_o^*)^2 + (a^* - a_o^*)^2 + (b^* - b_o^*)^2}$$

Dans cette équation, AE représente la différence de couleur entre deux mèches, (dans le cas présent la montée de la coloration), L* , a* , et b* représentent respectivement l'intensité, la nuance et la saturation de la mèche teinte, $L_o$* , $a_o$* et $b_o$* représentant respectivement l'intensité, la nuance et la saturation de la mèche témoin non teinte.
Plus la valeur de ΔE est importante, plus la différence de couleur entre les deux mèches est importante, et dans le cas présent, plus la montée de la coloration est importante et donc plus la teinture est puissante.

Les résultats ont été réunis dans le tableau (I) ci-dessous.

Tableau (I)

| Composition A (art antérieur) | |
|---|---|
| Paratoluènediamine | 0,122 g |
| 3,6-diméthyl-1 H-pyrazolo [5,1-c][1, 2, 4] triazole | 0,136 g |
| Aculyn 22 (Rohm and Haas) | 0,75 g MA* |
| Laccase issue de Trametes Versicolor | $10.10^6$ unités U |
| Agent de pH | q.s.p. pH 7 |
| Eau déminéralisée q.s.p. | 100 g |
| Composition B (invention) | |
| Paratoluènediamine | 0,122 g |
| 3,6-diméthyl-1 H-pyrazolo [5,1-c][1, 2, 4] triazole | 0,136 g |
| N, O-carboxyméthyl chitosan - Chitogenics ltd | 0,75 g MA* |
| Laccase issue de Trametes Versicolor | $10.10^6$ unités U |
| Agent de pH | q.s.p. ph 7 |
| Eau déminéralisée q.s.p. | 100 g |

* désigne Matière Active

| Composition | L* | a* | b* | Montée de la coloration |
|---|---|---|---|---|
| **A**(art antérieur) | 36,58 | 21,28 | 5,45 | 29,75 |
| **B** (invention) | 33,50 | 24,28 | 5,55 | 32,57 |
| Témoin (non coloré) | 57,20 | 1,07 | 11,64 | |

Ces résultats démontrent que la teinture contenant du N,O-carboxyméthylchitosane (selon l'invention) est plus puissante que celle contenant de l'Aculyn 22 (art antérieur).

EXEMPLE 2

On a étudié la conservation de l'activité enzymatique d'une laccase, d'une part, dans un milieu contenant un épaississant de l'art antérieur, l'ACULYN 22, et d'autre part, dans le milieu selon la présente invention, c'est-à-dire avec un N,O-carboxyméthylchitosane.
On a ainsi comparé une composition de l'art antérieur A à une composition selon l'invention B.

| Composition A (art antérieur) | |
|---|---|
| Aculyn 22 (Rohm and Haas ) | 0,75 g MA * |
| Laccase issue de Trametes Versicolor | $10.10^6$ unitésU |
| Agent de pH | q.s.p. pH 7 |
| Eau déminéralisée q.s.p. | 100 g |

* désigne Matière Active

| Composition B (invention) | |
|---|---|
| N,O-carboxyméthyl chitosan - Chitogenics ltd | 0,75 g MA* |
| Laccase SP809 - Novo Nordisk | $10.10^6$ unitésU |

(suite)

| Composition B (invention) | |
|---|---|
| Agent de pH | q.s.p. pH 7 |
| Eau déminéralisée q.s.p. | 100 g |

Les résultats ont été réunis sur la courbe du tableau (II) suivant :

**Tableau (II)**

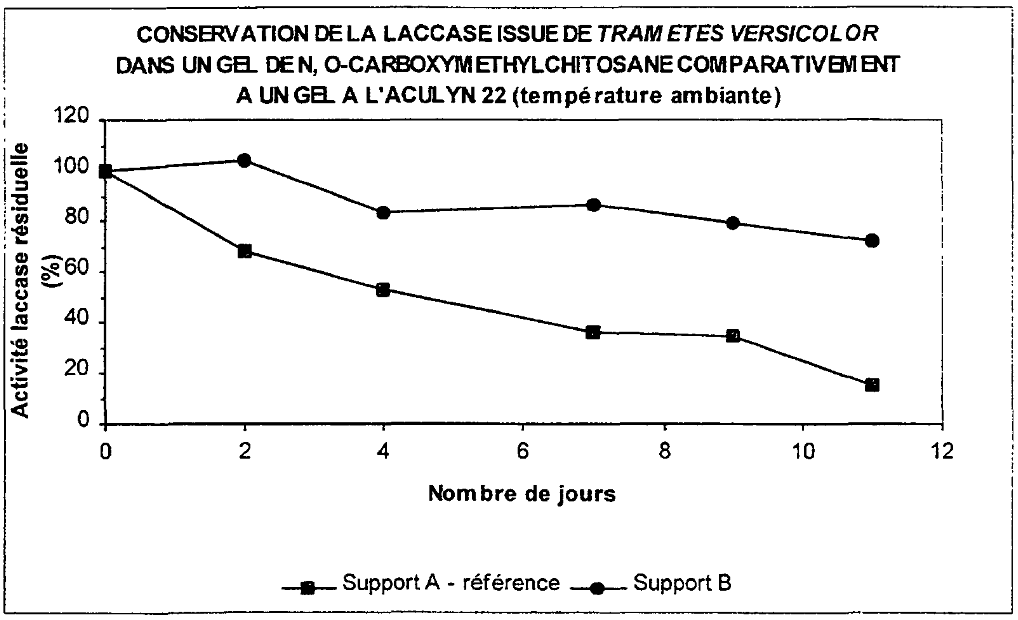


Ces résultats démontrent que la laccase présente une conservation bien supérieure dans un milieu au N,O-carboxyméthylchitosane (milieu selon l'invention avec le support B).

## Revendications

**1.** Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :

- au moins un colorant d'oxydation ,
- au moins une enzyme de type oxydo-réductase à 2 électrons en présence d'un donneur pour ladite enzyme ou à 4 électrons , et
- au moins un chitosane salifié par un acide organique ou minéral, permettant d'obtenir à 1 % dans l'eau une solution visuellement limpide,
  ou un N,O-carboxyméthylchitosane
- ou un chitosane chimiquement modifié comprenant un ou plusieurs motifs de formule (I) suivante :

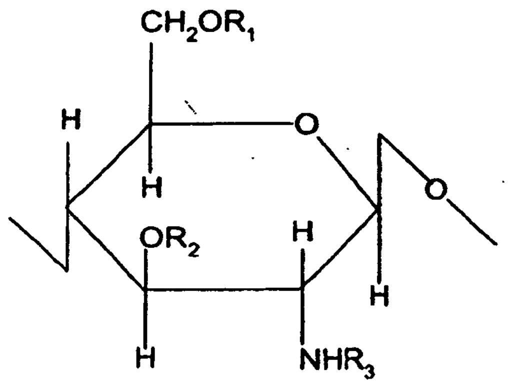

(I)

dans laquelle,

$R_1$, $R_2$, identiques ou différents, désignent un atome d'hydrogène ou un radical -XCOOM,

$R_3$ désigne un atome d'hydrogène, un radical $-COCH_3$, ou un radical -CO-X-COOM,

X représente un radical alkylène en $C_1$-$C_8$ éventuellement ramifié, ou substitué par un ou plusieurs groupements hydroxyle, halogène ou époxy,

M désigne un atome d'hydrogène ou un cation choisi parmi les métaux alcalins, alcalino-terreux, l'ammonium, une amine ou une alcanolamine organiques,

sous réserve que, l'un au moins des motifs de formule (I) comportant un radical $R_1$ et/ou $R_2$ et/ou $R_3$ désigne -XCOOM et/ou un radical $R_3$ désigne -CO-X-COOM.

2. Composition selon la revendication 1, **caractérisée par le fait que** le chitosane salifié par un acide organique est un pyrrolidone carboxylate de chitosane.

3. Composition selon la revendication 1, **caractérisée par le fait que** le chitosane chimiquement modifié de formule (I) est un N,O-carboxyméthylchitosane, un N-carboxybutylchitosane, un N-succinylchitosane, un N-succinylcarboxyméthylchitosane.

4. Composition selon la revendication 3, **caractérisée par le fait que** le chitosane chimiquement modifié de formule (I) est le N,O-carboxyméthylchitosane.

5. Composition selon l'une quelconque des revendications précédentes, **caractérisée par le fait que** le ou les chitosanes salifiés ou modifiés de formule (I) représentent de 0,01 à 20% en poids du poids total de la composition tinctoriale prête à l'emploi.

6. Composition selon la revendication 5, **caractérisée par le fait que** le ou les chitosanes salifiés ou modifiés de formule (I) représentent de 0,1 à 5% en poids du poids total de la composition tinctoriale prête à l'emploi.

7. Composition selon la revendication 1, **caractérisée par le fait que** les oxydo-réductases à 2 électrons sont utilisées avec un donneur pou la ou lesdites enzyme et sont choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

8. Composition selon la revendication 1, **caractérisée par le fait que** les oxydo-réductases à 4 électrons sont choisies parmi les laccases, les tyrosinases, les catéchol oxydases et les polyphénols oxydases.

9. Composition selon la revendication 8, **caractérisée par le fait que** les oxydo-réductases à 4 électrons sont choisies parmi les laccases d'origine végétale, d'origine animale, d'origine fongique ou d'origine bactérienne et parmi les laccases obtenues par biotechnologie.

**10.** Composition selon la revendication 9, **caractérisée par le fait que caractérisée par le fait que** la laccase est d'origine végétale et choisie parmi les laccases présentes dans les extraits d'Anacardiacées ; de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

**11.** Composition selon la revendication 8 ou 9, **caractérisée par le fait que** la laccase est d'origine fongique ou obtenue par biotechnologie.

**12.** Composition selon la revendication 11, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

**13.** Composition selon l'une quelconque des revendications précédentes, **caractérisée par le fait que** les oxydo-réductases à 2 électrons ou à 4 électrons représentent de 0,01 à 20 % en poids du poids total de la composition tinctoriale prête à l'emploi.

**14.** Composition selon la revendication 13, **caractérisée par le fait que** les oxydo-réductases à 2 électrons ou à 4 électrons représentent de 0,1 à 5 % en poids du poids total de la composition tinctoriale prête à l'emploi.

**15.** Composition selon l'une quelconque des revendications précédentes, **caractérisée par le fait que** le ou les colorants d'oxydation sont choisis parmi les bases d'oxydation et/ou les coupleurs.

**16.** Composition la revendication 15, **caractérisée par le fait que** les bases d'oxydation sont des paraphénylènediamines, des bases doubles, des para-aminophénols et des bases hétérocycliques, et leurs sels d'addition avec un acide.

**17.** Composition selon la revendication 16, **caractérisée par le fait que** les paraphénylènediamines sont choisies parmi celles de formule (II) suivante et leurs sels d'addition avec un acide :

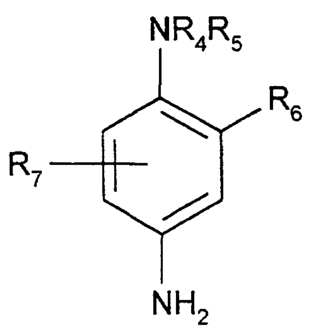

(II)

dans laquelle :

- $R_4$ et $R_5$, identiques ou différents, représentent un atome d'hydrogène, un radical alkyle en $C_1$-$C_4$, ou monohydroxyalkyle en $C_1$-$C_4$ ;
- $R_6$ représente un atome d'hydrogène ou d'halogène, un radical alkyle en $C_1$-$C_4$, ou monohydroxyalkyle en $C_1$-$C_4$ ;
- $R_7$ représente représentent un atome d'hydrogène ou un radical alkyle en $C_1$-$C_4$ .

**18.** Composition selon la revendication 17, **caractérisée par le fait que** les paraphénylènediamines de formule (II) sont choisies parmi :

la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxy-éthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, et leurs sels d'addition avec un acide.

**19.** Composition selon la revendication 16, **caractérisée par le fait que** les bases doubles sont choisies parmi les composés de formule (III) suivante, et leurs sels d'addition avec un acide :

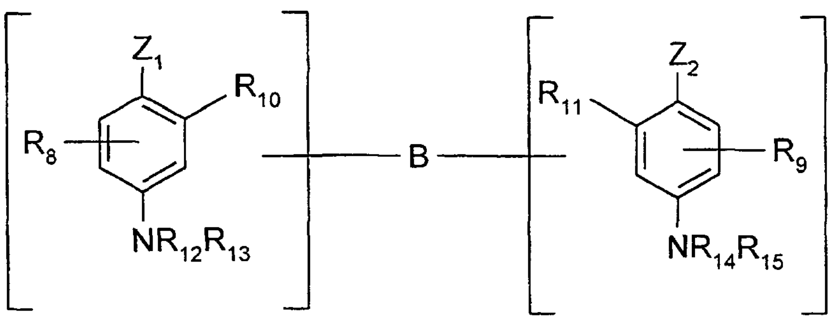

(III)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y;
- le bras de liaison B représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_8$ et $R_9$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{10}$, $R_{11}$, $R_{12}$, $R_{13}$, $R_{14}$ et $R_{15}$, identiques ou différents, représentent un atome d'hydrogène, un bras de liaison B ou un radical alkyle en $C_1$-$C_4$ ;

étant entendu que les composés de formule (III) ne comportent qu'un seul bras de liaison B par molécule.

**20.** Composition selon la revendication 19, **caractérisée par le fait que** les bases doubles de formule (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

**21.** Composition selon la revendications 16, **caractérisée par le fait que** les para-aminophénols sont choisis parmi ceux de formule (IV) suivante et leurs d'addition avec un acide :

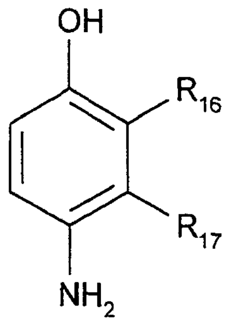

(IV)

dans laquelle :

- $R_{16}$ et $R_{17}$, identiques ou différents, représentent un atome d'hydrogène ou d'halogène, un radical alkyle en $C_1$-$C_4$, monohydroxyalkyle en $C_1$-$C_4$, alcoxy($C_1$-$C_4$)alkyle en $C_1$-$C_4$, aminoalkyle en $C_1$-$C_4$, monohydroxyalkyle($C_1$-$C_4$)aminoalkyle en $C_1$-$C_4$,

étant entendu qu'au moins un des radicaux $R_{16}$ et $R_{17}$ représente un atome d'hydrogène .

**22.** Composition selon la revendication 21, **caractérisée par le fait que** les para-aminophénols de formule (IV) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

**23.** Composition selon la revendication 16, **caractérisée par le fait que** les bases hétérocycliques sont choisies parmi les dérivés pyridiniques, les dérivés pyrimidiniques, les dérivés pyrazoliques, et leurs sels d'addition avec un acide.

**24.** Composition selon la revendication 15, **caractérisée par le fait que** les coupleurs sont des méta-aminophénols, des métaphénylènediamines, des métadiphénols, ou des coupleurs hétérocycliques, et leurs sels d'addition avec un acide.

**25.** Composition selon la revendication 24, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

**26.** Composition selon l'une quelconque des revendications 15 à 25, **caractérisée par le fait que** le ou les colorants d'oxydation (bases et/ou coupleurs) représentent de 0,001 à 20 % en poids du poids total de la composition tinctoriale prête à l'emploi.

**27.** Composition selon la revendication 26, **caractérisée par le fait que** le ou les colorants d'oxydation représentent de 0,01 à 10 % en poids du poids total de la composition tinctoriale prête à l'emploi.

**28.** Composition selon l'une quelconque des revendications précédentes, **caractérisée par le fait qu'**elle renferme un ou plusieurs colorants directs.

**29.** Composition selon l'une quelconque des revendications précédentes, **caractérisée par le fait qu'**elle présente un pH compris entre 3 et 11.

**30.** Composition selon l'une quelconque des revendications précédentes, **caractérisée par le fait que** le milieu approprié pour la teinture est l'eau ou un mélange d'eau et d'au moins un solvant organique.

**31.** Procédé de teinture d'oxydation des fibres kératiniques et en particulier des fibres kératiniques humaines telles que les cheveux, **caractérisé par le fait qu'**on applique sur lesdites fibres, à une température d'application comprise entre la température ambiante et 80°C, au moins une composition tinctoriale prête à l'emploi telle que définie dans l'une quelconque des revendications précédentes, pendant un temps suffisant pour développer la coloration désirée.

**32.** Procédé selon la revendication 31, **caractérisé par le fait que** la température d'application est comprise entre la température ambiante et 50°C.

**33.** Procédé selon la revendication 31 ou 32, **caractérisé par le fait que** le temps suffisant au développement de la coloration est compris entre 1 et 60 minutes.

**34.** Procédé selon la revendication 33, **caractérisé par le fait que** le temps suffisant au développement de la coloration est compris entre 5 et 30 minutes.

**35.** Procédé selon l'une quelconque des revendications 31 à 34, **caractérisé par le fait qu'**il comporte une étape préliminaire consistant à stocker sous forme séparée, d'une part, une composition (A) comprenant, dans un milieu approprié pour la teinture, au moins un colorant d'oxydation tel que défini à l'une quelconque des revendications 1 et 15 à 27, et d'autre part, une composition (B) renfermant, dans un milieu approprié pour la teinture, au moins une enzyme de type oxydo-réductase à 2 ou à 4 électrons, ladite composition (A) et/ou ladite composition (B) renfermant au moins un chitosane salifié ou chimiquement modifié tel que défini à l'une quelconque des revendications 1 à 6, puis à procéder à leur mélange au moment de l'emploi avant d'appliquer ce mélange sur les fibres kératiniques.

**36.** Dispositif de teinture à plusieurs compartiments, **caractérisé par le fait qu'**il comporte au moins un premier compartiment renfermant la composition (A) telle que définie dans la revendication 35 et au moins un second compartiment renfermant la composition (B) telle que définie dans la revendication 35.

## Patentansprüche

**1.** Gebrauchsfertige Zusammensetzung zum oxidativen Färben von Keratinfasern und insbesondere menschlichen Keratinfasern, wie zum Färben der Haare, **dadurch gekennzeichnet, dass** sie in einem zum Färben geeigneten Medium enthält:

- mindestens einen Oxidationsfarbstoff,
- mindestens ein Enzym vom Typ der Oxidoreduktasen, die 2 Elektronen übertragen, in Gegenwart eines Donors für das Enzym oder vom Typ der Oxidoreduktasen, die 4 Elektronen übertragen,
- mindestens ein Chitosan, das mit einer organischen oder anorganischen Säure in das Salz überführt wurde und mit dem bei einer Konzentration von 1 % in Wasser eine für das Auge klare Lösung erhalten werden kann, oder ein N,O-carboxymethylchitosan,
- oder ein chemisch modifiziertes Chitosan, das eine oder mehrere Einheiten der folgenden Formel (I) enthält:

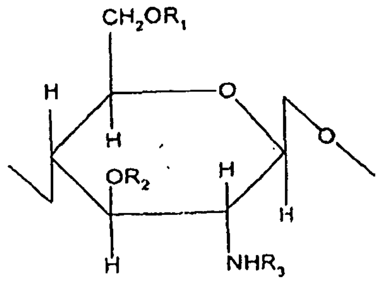

**(I)**

worin bedeuten:

die Gruppen $R_1$ und $R_2$, die gleich oder verschieden sind, ein Wasserstoffatom oder eine Gruppe -XCOOM, die Gruppe $R_3$ ein Wasserstoffatom, die Gruppe $-COCH_3$ oder eine Gruppe -CO-X-COOM,

die Gruppe X eine $C_{1-8}$-Alkylengruppe, die gegebenenfalls verzweigt ist oder mit einer oder mehreren Gruppen Hydroxy, Halogen oder Epoxy substituiert ist,
M ein Wasserstoffatom oder ein Kation, das unter den Alkalimetallen, Erdalkalimetallen, Ammonium, Aminen oder organischen Alkanolaminen ausgewählt ist,

mit der Maßgabe, dass mindestens eine der Einheiten der Formel (I), die eine Gruppe $R_1$ und/oder $R_2$ und/oder $R_3$ aufweist, -XCOOM und/oder eine Gruppe $R_3$ -CO-X-COOM bedeutet.

**2.** Zusammensetzung nach Anspruch 1, **dadurch gekennzeichnet, dass** das mit einer organischen Säure in das Salz überführte Chitosan ein Chitosanpyrrolidonchitosan ist.

**3.** Zusammensetzung nach Anspruch 1, **dadurch gekennzeichnet, dass** das chemisch modifizierte Chitosan der Formel (I) ein N,O-Carboxymethylchitosan, N-Carboxybutylchitosan, N-Succinylchitosan oder N-Succinylcarboxymethylchitosan ist.

**4.** Zusammensetzung nach Anspruch 3, **dadurch gekennzeichnet, dass** das chemisch modifizierte Chitosan der Formel (I) das N,O-Carboxymethylchitosan ist.

**5.** Zusammensetzung nach einem der vorhergehenden Ansprüche, **dadurch gekennzeichnet, dass** das oder die in das Salz überführte(n) oder modifizierte(n) Chitosan(e) der Formel (I) 0,01 bis 20 Gew.-% des Gesamtgewichts der gebrauchsfertigen Farbmittelzusammensetzung ausmachen.

**6.** Zusammensetzung nach Anspruch 5, **dadurch gekennzeichnet, dass** das oder die in das Salz überführte(n) oder modifizierte(n) Chitosan(e) der Formel (I) 0,1 bis 5 Gew.-% des Gesamtgewichts der gebrauchsfertigen Farbmittelzusammensetzung ausmachen.

**7.** Zusammensetzung nach Anspruch 1, **dadurch gekennzeichnet, dass** die Oxidoreduktasen, die 2 Elektronen übertragen, zusammen mit einem Donor für das Enzym oder die Enzyme verwendet werden und unter den Pyranose-Oxidasen, Glucose-Oxidasen, Glycerin-Oxidasen, Lactat-Oxidasen, Pyruvat-Oxidasen, Uricasen, Cholin-Oxidasen, Sarcosin-Oxidasen, Bilirubin-Oxidasen und Aminosäure-Oxidasen ausgewählt sind.

**8.** Zusammensetzung nach Anspruch 1, **dadurch gekennzeichnet, dass** die Oxidoreduktasen, die 4 Elektronen übertragen, unter den Laccasen, Tyrosinasen, Catechin-Oxidasen und Polyphenol-Oxidasen ausgewählt sind.

**9.** Zusammensetzung nach Anspruch 8, **dadurch gekennzeichnet, dass** die Oxidoreduktasen, die 4 Elektronen übertragen, unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

**10.** Zusammensetzung nach Anspruch 9, **dadurch gekennzeichnet, dass** die Laccase pflanzlicher Herkunft ist und unter den Laccasen ausgewählt ist, die in Extrakten von Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica und Pistacia palaestina enthalten sind.

**11.** Zusammensetzung nach Anspruch 8 oder 9, **dadurch gekennzeichnet, dass** die Laccase von Pilzen stammt oder biotechnologisch hergestellt ist.

**12.** Zusammensetzung nach Anspruch 11, **dadurch gekennzeichnet, dass** die Laccase unter den Laccasen ausgewählt ist, die von Polyporus versicolor, Rhizoctonia praticola, Rhus vernicifera, Scytalidium, Polyporus pinsitus, Myceliophtora thermophila, Rhizoctonia solani, Pyricularia oryzae, Trametes versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

**13.** Zusammensetzung nach einem der vorhergehenden Ansprüche, **dadurch gekennzeichnet, dass** die Oxidoreduktasen, die 2 oder 4 Elektronen übertragen, 0,01 bis 20 Gew.-% des Gesamtgewichts der gebrauchsfertigen Farbmittelzusammensetzung ausmachen.

**14.** Zusammensetzung nach Anspruch 13, **dadurch gekennzeichnet, dass** die Oxidoreduktasen, die 2 oder 4 Elektronen übertragen, 0,1 bis 5 Gew.-% des Gesamtgewichts der gebrauchsfertigen Farbmittelzusammensetzung ausmachen.

**15.** Zusammensetzung nach einem der vorhergehenden Ansprüche, **dadurch gekennzeichnet, dass** der oder die Oxidationsfarbstoff(e) unter den Oxidationsbasen und/oder Kupplern ausgewählt sind.

**16.** Zusammensetzung nach Anspruch 15, **dadurch gekennzeichnet, dass** die Oxidationsbasen unter den p-Phenylendiaminen, Doppelbasen, p-Aminophenolen und den heterocyclischen Basen und ihren Additionssalzen mit einer Säure ausgewählt sind.

**17.** Zusammensetzung nach Anspruch 16, **dadurch gekennzeichnet, dass** die p-Phenylendiamine unter den Verbindungen der folgenden Formel (II) und deren Additionssalzen mit einer Säure ausgewählt sind:

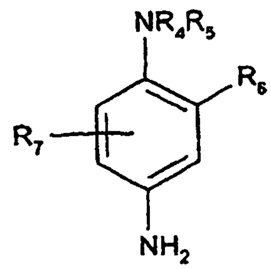

(II)

worin bedeuten:

- $R_4$ und $R_5$, die gleich oder verschieden sind, ein Wasserstoffatom, $C_{1-4}$-Alkyl oder $C_{1-4}$-Monohydroxyalkyl,
- $R_6$ ein Wasserstoff- oder Halogenatom, $C_{1-4}$-Alkyl oder $C_{1-4}$-Monohydroxyalkyl,
- $R_7$ Wasserstoff oder $C_{1-4}$-Alkyl.

**18.** Zusammensetzung nach Anspruch 17, **dadurch gekennzeichnet, dass** die p-Phenylendiamine der Formel (II) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-bis(β-Hydroxyethyl)amino-2-methylanilin, 4-N,N-bis(β-Hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin und deren Additionssalzen mit einer Säure ausgewählt sind.

**19.** Zusammensetzung nach Anspruch 16, **dadurch gekennzeichnet, dass** die Doppelbasen unter den Verbindungen der folgenden Formel (III) und deren Additionssalze mit einer Säure ausgewählt sind:

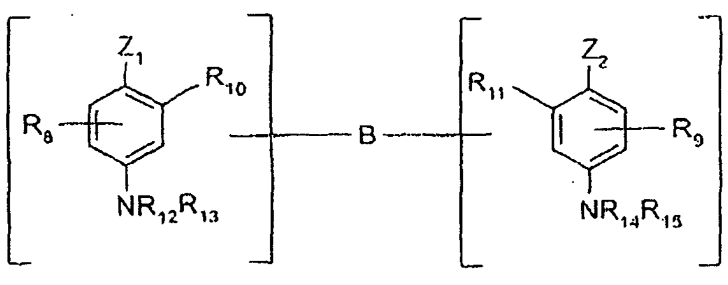

(III)

worin bedeuten:

- $Z_1$ und $Z_2$, die identisch oder voneinander verschieden sind, Hydroxy oder eine $NH_2$-Gruppe, die mit $C_{1-4}$-Alkyl oder einer Verbindungsgruppe Y substituiert sein kann,
- die Verbindungsgruppe B eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder $C_{1-6}$-Alkoxygruppen substituiert sein kann,
- $R_8$ und $R_9$ ein Wasserstoff- oder Halogenatom, $C_{1-4}$-Alkyl, $C_{1-4}$-Monohydroxyalkyl, $C_{2-4}$-Polyhydroxyalkyl, $C_{1-4}$-Aminoalkyl oder eine Verbindungsgruppe Y,
- $R_{10}$, $R_{11}$, $R_{12}$, $R_{13}$, $R_{14}$ und $R_{15}$, die identisch oder voneinander verschieden sind, Wasserstoff, eine Verbindungsgruppe B oder $C_{1-4}$-Alkyl,

mit der Maßgabe, dass die Verbindungen der Formel (III) nur eine Verbindungsgruppe B pro Molekül aufweisen.

**20.** Zusammensetzung nach Anspruch 19, **dadurch gekennzeichnet, dass** die Doppelbasen der Formel (III) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin, 1,8-Bis(2,5-diamino-phenoxy)-3,5-dioxaoctan und deren Additionssalzen mit einer Säure ausgewählt sind.

**21.** Zusammensetzung nach Anspruch 16, **dadurch gekennzeichnet, dass** die p-Aminophenole unter den Verbindungen der folgenden Formel (IV) und deren Additionssalze mit einer Säure ausgewählt sind:

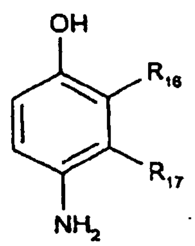

(IV)

worin bedeuten:

- $R_{16}$ und $R_{17}$, die gleich oder verschieden sind, ein Wasserstoff- oder Halogenatom, $C_{1-4}$-Alkyl, $C_{1-4}$-Monohydroxyalkyl, $C_{1-4}$-Alkoxy-$C_{1-4}$-alkyl, $C_{1-4}$-Aminoalkyl oder $C_{1-4}$-MonoHydroxyalkyl-$C_{1-4}$-aminoalkyl,

mit der Maßgabe, dass mindestens eine der Gruppen $R_{16}$ oder $R_{17}$ Wasserstoff bedeutet.

**22.** Zusammensetzung nach Anspruch 21, **dadurch gekennzeichnet, dass** die p-Aminophenole der Formel (IV) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylamino-methyl)-phenol, 4-Amino-2-fluor-phenol und deren Additionssalzen mit einer Säure ausgewählt sind.

**23.** Zusammensetzung nach Anspruch 16, **dadurch gekennzeichnet, dass** die heterocyclischen Basen unter den Pyridinderivaten, Pyrimidinderivaten, Pyrazolderivaten und deren Additionssalzen mit einer Säure ausgewählt sind.

**24.** Zusammensetzung nach Anspruch 15, **dadurch gekennzeichnet, dass** die Kuppler unter den m-Aminophenolen, m-Phenylendiaminen, m-Dihydroxybenzolen oder den heterocyclischen Kupplern und deren Additionssalzen mit einer Säure ausgewählt sind.

**25.** Zusammensetzung nach Anspruch 24, **dadurch gekennzeichnet, dass** die Kuppler unter 2-Methyl-5-amino-phenol, 5-N-(β-Hydroxyethyl)-amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diaminobenzol, 1,3-Bis(2,4-diaminophenoxy)-propan, Sesamol, 1-Amino-2-methoxy-4,5-methylendioxy-benzol, α-Naphthol, 6-Hydroxyindol, 4-Hydroxyindol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on und den Additionssalzen dieser Verbindungen mit einer Säure ausgewählt sind.

**26.** Zusammensetzung nach einem der Ansprüche 15 bis 25, **dadurch gekennzeichnet, dass** der oder die Oxidationsfarbstoff(e) (Basen und/oder Kuppler) 0,001 bis 20 Gew.-% des Gesamtgewichts der gebrauchsfertigen Farbmittelzusammensetzung ausmachen.

**27.** Zusammensetzung nach Anspruch 26, **dadurch gekennzeichnet, dass** der oder die Oxidationsfarbstoff(e) 0,01 bis 10 Gew.-% des Gesamtgewichts der gebrauchsfertigen Farbmittelzusammensetzung ausmachen.

**28.** Zusammensetzung nach einem der vorhergehenden Ansprüche, **dadurch gekennzeichnet, dass** sie einen oder mehrere Direktfarbstoffe enthält.

**29.** Zusammensetzung nach einem der vorhergehenden Ansprüche, **dadurch gekennzeichnet, dass** sie einen pH-Wert im Bereich von 3 bis 11 aufweist.

**30.** Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

**31.** Verfahren zum oxidativen Färben von Keratinfasern und insbesondere menschlichen Keratinfasern, wie zum Färben der Haare, **dadurch gekennzeichnet, dass** auf die Fasern bei einer Applikationstemperatur im Bereich von Raumtemperatur bis 80 °C mindestens eine gebrauchsfertige Farbmittelzusammensetzung nach einem der vorhergehenden Ansprüche während einer Zeitspanne, die zur Entwicklung der gewünschten Färbung ausreichend ist, aufgebracht wird.

**32.** Verfahren nach Anspruch 31, **dadurch gekennzeichnet, dass** die Applikationstemperatur im Bereich von Raumtemperatur bis 50 °C liegt.

**33.** Verfahren nach Anspruch 31 oder 32, **dadurch gekennzeichnet, dass** die Zeitspanne, die zur Entwicklung der gewünschten Färbung ausreichend ist, im Bereich von 1 bis 60 min liegt.

**34.** Verfahren nach Anspruch 33, **dadurch gekennzeichnet, dass** die Zeitspanne, die zur Entwicklung der gewünschten Färbung ausreichend ist, im Bereich von 5 bis 30 min liegt.

**35.** Verfahren nach einem der Ansprüche 31 bis 34, **dadurch gekennzeichnet, dass** es einen vorbereitenden Schritt umfasst, der darin besteht, einerseits eine Zusammensetzung (A), die in einem zum Färben geeigneten Medium mindestens einen Oxidationsfarbstoff, wie er in den Ansprüchen 1 und 15 bis 27 definiert wurde, enthält, und andererseits eine Zusammensetzung (B) getrennt voneinander aufzubewahren, die in einem zum Färben geeigneten Medium mindestens ein Enzym vom Typ der Oxidoreduktasen, die 2 oder 4 Elektronen übertragen, enthält, wobei die Zusammensetzung (A) und/ oder die Zusammensetzung (B) mindestens ein in ein Salz überführtes oder chemisch modifiziertes Chitosan, wie es in den Ansprüchen 1 bis 6 definiert wurde, enthalten, und diese bei der Anwendung zu vermischen, bevor das Gemisch auf die Keratinfasern aufgebracht wird.

**36.** Vorrichtung mit mehreren Abteilungen, **dadurch gekennzeichnet, dass** sie mindestens eine erste Abteilung mit der in Anspruch 35 definierten Zusammensetzung (A) und mindestens eine zweite Abteilung mit der in Anspruch 35 definierten Zusammensetzung (B) enthält.

## Claims

**1.** Ready-to-use composition for the oxidation dyeing of keratin fibres, and in particular of human keratin fibres such as the hair, **characterized in that** it comprises, in a medium which is suitable for dyeing:

- at least one oxidation dye,

- at least one enzyme of 2-electron oxidoreductase type in the presence of a donor for the said enzyme or of 4-electron oxidoreductase type, and
- at least one chitosan salified with an organic or inorganic acid, allowing a visually clear solution to be obtained at a concentration of 1% in water,
  or an N,O-carboxymethylchitosan,
- or a chemically modified chitosan comprising one or more units of formula (I) below:

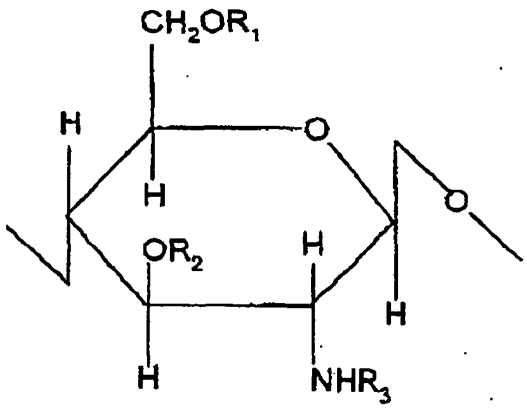

(I)

in which,

$R_1$ and $R_2$, which may be identical or different, denote a hydrogen atom or a radical -XCOOM,
$R_3$ denotes a hydrogen atom, a $-COCH_3$ radical or a radical -CO-X-COOM,
X represents a $C_1$-$C_8$ alkylene radical, which is optionally branched, or substituted with one or more hydroxyl, halogen or epoxy groups,
M denotes a hydrogen atom or a cation chosen from alkali metals, alkaline-earth metals, ammonium, an organic amine or an organic alkanolamine,

with the proviso that at least one of the units of formula (I) comprising a radical $R_1$ and/or $R_2$ and/or $R_3$ denotes -XCOOM and/or a radical $R_3$ denotes -CO-X-COOM.

2. Composition according to Claim 1, **characterized in that** the chitosan salified with an organic acid is a chitosan pyrrolidonecarboxylate.

3. Composition according to Claim 1, **characterized in that** the chemically modified chitosan of formula (I) is an N, O-carboxymethylchitosan, an N-carboxybutylchitosan, an N-succinylchitosan or an N-succinylcarboxymethylchitosan.

4. Composition according to Claim 3, **characterized in that** the chemically modified chitosan of formula (I) is N,O-carboxymethylchitosan.

5. Composition according to any one of the preceding claims, **characterized in that** the salified or modified chitosan (s) of formula (I) represent(s) from 0.01 to 20% by weight relative to the total weight of the ready-to-use dye composition.

6. Composition according to Claim 5, **characterized in that** the salified or modified chitosan(s) of formula (I) represent (s) from 0.1 to 5% by weight relative to the total weight of the ready-to-use dye composition.

7. Composition according to Claim 1, **characterized in that** the 2-electron oxidoreductases are used with a donor for the said enzyme (s) and are chosen from pyranose oxidases, glucose oxidases, glycerol oxidases, lactate oxidases, pyruvate oxidases, uxicases, choline oxidases, sarcosine oxidases, bilirubin oxidases and amino acid oxidases.

8. Composition according to Claim 1, **characterized in that** the 4-electron oxidoreductases are chosen from laccases, tyrosinases, catechol oxidases and polyphenol oxidases.

**9.** Composition according to Claim 8, **characterized in that** the 4-electron oxidoreductases are chosen from laccases of plant origin, of animal origin, of fungal origin or of bacterial origin and from laccases obtained by biotechnology.

**10.** Composition according to Claim 9, **characterized in that** the laccase is of plant origin and is chosen from the laccases present in extracts of Anacardiacea plants; of Podocarpacea plants; of Rosmarinus off.; of Solanum tuberosum; of Iris sp.; of Coffea sp.; of Daucus carrota; of Vinca minor; of Persea americana; of Catharanthus roseus; of Musa sp.; of Malus pumila; of Gingko biloba; of Monotropa hypopithys (Indian pipe), of Aesculus sp.; of Acer pseudoplatanus; of Prunus persica and of Pistacia palaestina.

**11.** Composition according to Claim 8 or 9, **characterized in that** the laccase is of fungal origin or is obtained by biotechnology.

**12.** Composition according to Claim 11, **characterized in that** the laccase is chosen from laccases obtained from Polyporus versicolor, from Rhizoctonia praticola, from Rhus vernicifera, from Scytalidium, from Polyporus pinsitus, from Myceliophtora thermophila, from Rhizoctonia solani, from Pyricularia orizae, from Trametes versicolor, from Fomes fomentarius, from Chaetomium thermophile, from Neurospora crassa, from Colorius versicol, from Botrytis cinerea, from Rigidoporus lignosus, from Phellinus noxius, from Pleurotus ostreatus, from Aspergillus nidulans, from Podospora anserina, from Agaricus bisporus, from Ganoderma lucidum, from Glomerella cingulata, from Lactarius piperatus, from Russula delica, from Heterobasidion annosum, from Thelephora terrestris, from Cladosporium cladosporioides, from Cerrena unicolor, from Coriolus hirsutus, from Ceriporiopsis subvermispora, from Coprinus cinereus, from Panaeolus papilionaceus, from Panaeolus sphinctrinus, from Schizophyllum commune, from Dichomitius squalens, and from variants thereof.

**13.** Composition according to any one of the preceding claims, **characterized in that** the 2-electron or 4-electron oxidoreductases represent from 0.01 to 20% by weight relative to the total weight of the ready-to-use dye composition.

**14.** Composition according to Claim 13, **characterized in that** the 2-electron or 4-electron oxidoreductases represent from 0.1 to 5% by weight relative to the total weight of the ready-to-use dye composition.

**15.** Composition according to any one of the preceding claims, **characterized in that** the oxidation dye(s) is(are) chosen from oxidation bases and/or couplers.

**16.** Composition according to Claim 15, **characterized in that** the oxidation bases are para-phenylenediamines, double bases, para-aminophenols and heterocyclic bases, and the addition salts thereof with an acid.

**17.** Composition according to Claim 16, **characterized in that** the para-phenylenediamines are chosen from those of formula (II) below, and the addition salts thereof with an acid:

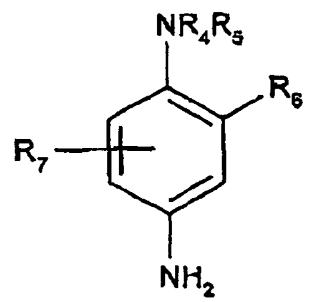

(II)

in which:

- $R_4$ and $R_5$, which may be identical or different, represent a hydrogen atom, a $C_1$-$C_4$ alkyl radical or a $C_1$-$C_4$ monohydroxyalkyl radical;
- $R_6$ represents a hydrogen or halogen atom, a $C_1$-$C_4$ alkyl radical or a $C_1$-$C_4$ monohydroxyalkyl radical;
- $R_7$ represents a hydrogen atom or a $C_1$-$C_4$ alkyl radical.

**18.** Composition according to Claim 17, **characterized in that** the para-phenylenediamines of formula (II) are chosen

from:

para-phenylenediamine, para-tolylenediamine, 2-chloro-para-phenylenediamine, 2,3-dimethyl-para-phenylene-diamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine,
4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine and N-ethyl-N-(β-hydroxyethyl)-para-phenylenediamine, and the addition salts thereof with an acid.

**19.** Composition according to Claim 16, **characterized in that** the double bases are chosen from the compounds of formula (III) below, and the addition salts thereof with an acid:

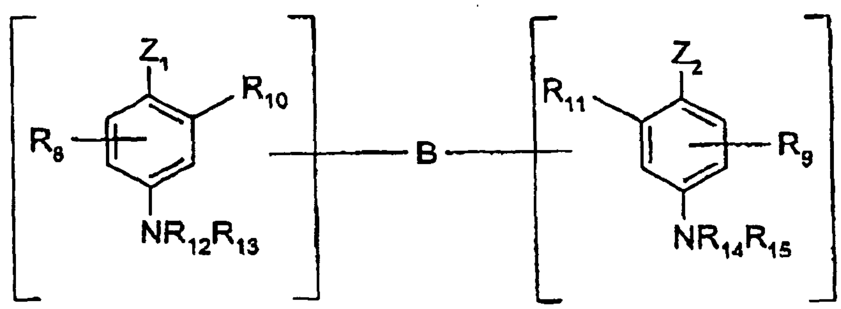

(III)

in which:

- $Z_1$ and $Z_2$, which may be identical or different, represent a hydroxyl or $-NH_2$ radical which can be substituted with a $C_1$-$C_4$ alkyl radical or with a linker arm Y;
- the linker arm B represents a linear or branched alkylene chain containing from 1 to 14 carbon atoms, which can be interrupted or end with one or more nitrogenous groups and/or with one or more hetero atoms such as oxygen, sulphur or nitrogen atoms, and optionally substituted with one or more hydroxyl or $C_1$-$C_6$ alkoxy radicals;
- $R_8$ and $R_9$ represent a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical or a linker arm Y;
- $R_{10}$, $R_{11}$, $R_{12}$, $R_{13}$, $R_{14}$ and $R_{15}$, which may be identical or different, represent a hydrogen atom, a linker arm B or a $C_1$-$C_4$ alkyl radical;

it being understood that the compounds of formula (III) comprise only one linker arm B per molecule.

**20.** Composition according to Claim 19, **characterized in that** the double bases of formula (III) are chosen from N, N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-bydroxyethyl)-N,N'-bis (4-aminophenyl)tetramethylenediamine, N,N'-bis(4-methylaminophenyl)tetramethylenediamine, N,N'-bis(ethyl) -N,N'-bis(4'-amino-3'-methyl-phexzyl)ethylenediamine and 1,8-bis(2,5-diaminophenoxy)-3,5-dioxaoctane, and the addition salts thereof with an acid.

**21.** Composition according to Claim 16, **characterized in that** the para-aminophenols are chosen from those of formula (IV) below, and the addition salts thereof with an acid:

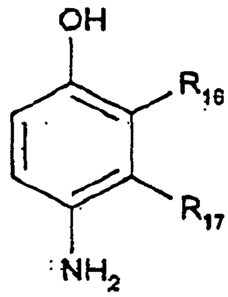

(IV)

in which:

- $R_{16}$ and $R_{17}$, which may be identical or different, represent a hydrogen or halogen atom, a $C_1$-$C_4$ alkyl radical, a $C_1$-$C_4$ monohydroxyalkyl radical, a ($C_1$-$C_4$)alkoxy($C_1$-$C_4$)alkyl radical, a $C_1$-$C_4$ aminoalkyl radical or a monohydroxy($C_1$-$C_4$)alkylamino($C_1$-$C_4$)alkyl radical,

it being understood that at least one of the radicals $R_{16}$ and $R_{17}$ represents a hydrogen atom.

**22.** Composition according to Claim 21, **characterized in that** the para-aminophenols of formula (IV) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethyl-aminomethyl)phenol, and 4-amino-2-fluorophenol, and the addition salts thereof with an acid.

**23.** Composition according to Claim 16, **characterized in that** the heterocyclic bases are chosen from pyridine derivatives, pyrimidine derivatives and pyrazole derivatives, and the addition salts thereof with an acid.

**24.** Composition according to Claim 15, **characterized in that** the couplers are meta-aminophenols, meta-phenylenediamines, meta-diphenols or heterocyclic couplers, and the addition salts thereof with an acid.

**25.** Composition according to Claim 24, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloxo-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, 1-amino-2-methoxy-4,5-methylenedioxybenzene, α-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one and 1-phenyl-3-methylpyrazol-5-one, and the addition salts thereof with an acid.

**26.** Composition according to any one of Claims 15 to 25, **characterized in that** the oxidation dye(s) (bases and/or couplers) represent(s) from 0.001 to 20% by weight relative to the total weight of the ready-to-use dye composition.

**27.** Composition according to Claim 26, **characterized in that** the oxidation dye(s) represent(s) from 0.01 to 10% by weight relative to the total weight of the ready-to-use dye composition.

**28.** Composition according to any one of the preceding claims, **characterized in that** it contains one or more direct dyes.

**29.** Composition according to any one of the preceding claims, **characterized in that** it has a pH of between 3 and 11.

**30.** Composition according to any one of the preceding claims, **characterized in that** the medium which is suitable for dyeing is water or a mixture of water and at least one organic solvent.

**31.** Process for the oxidation dyeing of keratin fibres, and in particular human keratin fibres such as the hair, **characterized in that** at least one ready-to-use dye composition as defined in any one of the preceding claims is applied to the said fibres, at an application temperature of between room temperature and 80°C, for a period which is sufficient to develop the desired coloration.

**32.** Process according to Claim 31, **characterized in that** the application temperature is between room temperature and 50°C.

**33.** Process according to Claim 31 or 32, **characterized in that** the time which is sufficient to develop the coloration is between 1 and 60 minutes.

**34.** Process according to Claim 33, **characterized in that** the time which is sufficient to develop the coloration is between 5 and 30 minutes.

**35.** Process according to any one of Claims 31 to 34, **characterized in that** it comprises a preliminary step which consists in separately storing, on the one hand, a composition (A) comprising, in a medium which is suitable for dyeing, at least one oxidation dye as defined in any one of Claims 1 and 15 to 27, and, on the other hand, a composition (B) containing, in a medium which is suitable for dyeing, at least one enzyme of 2- or 4-electron oxidoreductase type, the said composition (A) and/or the said composition (B) containing at least one salified or chemically modified chitosan as defined in any one of Claims 1 to 6, and then in mixing them together at the time of use, after which this mixture is applied to the keratin fibres.

**36.** Multi-compartment dyeing device, **characterized in that** it includes at least a first compartment containing composition (A) as defined in Claim 35 and at least a second compartment containing composition (B) as defined in Claim 35.